# EUROPEAN PATENT APPLICATION

(11) **EP 4 623 831 A1**
(43) Date of publication of application: **01.10.2025**
(21) Application number: 24167853.1
(22) Date of filing: 29.03.2024
(51) Int. Cl.: A61B 6/03, A61B 6/00, A61B 6/50

(54) **COMPUTED TOMOGRAPHY IMAGE ACQUISITION CONTROLLED BY MOTION SENSOR**

(71) Applicant: Koninklijke Philips N.V., 5656 AG Eindhoven (NL)
(72) Inventor: KOEHLER, Thomas, Eindhoven (NL); SENEGAS, Julien Thomas, Eindhoven (NL); BUELOW, Thomas, 5656AG Eindhoven (NL); NETSCH, Thomas, Eindhoven (NL)
(74) Representative: Philips Intellectual Property & Standards

(57) **Abstract**

The present invention relates to a method for adapting a parameter of an image acquisition with a computed tomography apparatus to a motion of a patient to be imaged. The method comprises the steps of receiving, with a motion sensor, measurement data about a movement of a patient to be imaged with the computed tomography apparatus, and determining, on the basis of the measurement data of the motion sensor, a motion of the patient. The method comprises further the steps of determining, in case the determined motion exceeds a predetermined threshold, an adapted value of the parameter of the image acquisition based on the determined motion, and acquiring an image of at least a region of the patient with the computed tomography apparatus utilizing the adapted value of the parameter of the image acquisition.

## Description

### FIELD OF THE INVENTION

The present invention relates to a method for adapting a parameter of an image acquisition with a computed tomography apparatus to a motion of a patient to be imaged, a device for adapting a parameter of an image acquisition with a computed tomography apparatus to a motion of a patient to be imaged, and a data processing apparatus.

### BACKGROUND OF THE INVENTION

A fair fraction of all computed tomography examinations in clinical practice are scans of the head. Among others, a computed tomography scan of the head is indicated after a head injury or if there is suspicion for stroke. Giving this initial indication and as the patients may be nervous or have pain, it appears that patients may often be not fully cooperative, as they may not be able to accurately follow the instructions of the operator of the computed tomography apparatus not to move the head, for example. However, as computed tomography examinations of the head have rather demanding image quality requirements, scans are usually done at rather limited collimation of about 2 cm or 4 cm of collimation size. This implies that the anatomical region of interest cannot be covered by a single axial scan and either a step-and-shoot or a helical acquisition is needed. In the presence of patient motion, often a helical acquisition at large pitch is selected in order to minimize the total scan time. However, a step-and-shoot acquisition may become more relevant in the future, although there may be still motion of the patient between the individual axial acquisitions present.

The inventors of the present invention have thus found that it would be advantageous to have a method that recognizes motion of a patient during imaging with a computed tomography apparatus or between individual image frames that at least partially overcomes the drawbacks of the prior art and adapts the image acquisition based on the detected motion of the patient.

### SUMMARY OF THE INVENTION

It is an object of the present invention to provide an improved method for adapting a parameter of an image acquisition with a computed tomography apparatus to a motion of a patient to be imaged.

The object of the present invention is solved by the subject matter of the independent claims, wherein further embodiments are incorporated in the dependent claims.

The described embodiments similarly pertain to the method for adapting a parameter of an image acquisition with a computed tomography apparatus to a motion of a patient to be imaged, the device for adapting a parameter of an image acquisition with a computed tomography apparatus to a motion of a patient to be imaged, and the data processing apparatus. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a method for adapting a parameter of an image acquisition with a computed tomography apparatus to a motion of a patient to be imaged. The method comprises the steps of receiving, with a motion sensor, measurement data about a movement of a patient to be imaged with the computed tomography apparatus, determining, on the basis of the measurement data of the motion sensor, a motion of the patient, determining, in case the determined motion exceeds a predetermined threshold, an adapted value of the parameter of the image acquisition based on the determined motion, and acquiring an image of at least a region of the patient with the computed tomography apparatus utilizing the adapted value of the parameter of the image acquisition.

Thus, in an image acquisition process with a computed tomography apparatus, the motion of the patient to be imaged is detected with a motion sensor. Based on the detected motion, it may be determined whether the occurred movement of the patient is detrimental for the result of an image reconstruction. In case the determined motion exceeds a predetermined threshold, it may be determined how the image acquisition process can be adapted to the occurred motion of the patient. In order to adapt the image acquisition process and compensate for the movement, an adapted value of a parameter of the image acquisition is determined, such that the image acquisition with the computed tomography apparatus can proceed with the adapted value, thus compensating for the movement of the patient.

Specifically, in a preparation phase before the image acquisition starts, the motion sensor can track the movement of the patient's body or the head and can derive typical motion parameters for nodding and shaking of the head. Based on a geometrical model of the computed tomography system geometry, safety margins can be calculated and the distance between axial scans can be planned to have a patient-specific appropriate overlap that will ensure that no gaps in image acquisition occur.

As another example, during the scan, the motion sensor can track the actual position of the scan with respect to the patient's body for each axial scan. If a severe gap to a preceding axial scan is detected, the present scan can be repeated. If a rotation, i.e. around an axis different from the rotation axis of the CT system, or shift, i.e. along the rotation axis of the CT system, of the body part of the patient like the head in the current axial scan is detected, the couch travel to a next axial scan can be reduced to avoid a gap to the subsequent axial scan.

In addition, the detected motion on the basis of the measurement data of the motion sensor can be provided and used by an image reconstruction algorithm during the reconstruction phase to compensate for the detected motion. The reconstruction can then compensate for the movement like a tilt or a shift during back-projection and ensure that the images from the different axial acquisitions are reconstructed properly in the head's coordinate system to allow for an easy reading of the volume.

In addition or alternatively, each projection data can be geometrically corrected to compensate the detected movements. A full reconstruction can then be performed from a set of projections that are geometrically distributed in a non-standard way, e.g. by iterative reconstruction techniques such as algebraic reconstruction.

Further, data from the motion sensor can be used to trigger axial image acquisitions at a point of no or little motion.

Thus, the present invention relates to a method that uses a motion sensor to control and adapt at least one parameter of the image acquisition to compensate for possible detrimental effects on the reconstructed image based on patient motion. The appearance of motion artefacts can be reduced by using a motion sensor that triggers the image acquisition once it detects a period of no or little motion and/or computes the head motion and applies the motion parameters to steer and adapt the image acquisition and/or the reconstruction process. In combination with a step-and-shoot acquisition with axial scans or a helical acquisition, motion artefacts can be reduced efficiently by triggering each axial image acquisition independently by applying the proposed motion correction method of the present invention.

In an embodiment of the invention, the image acquisition is a step-and-shoot image acquisition. In this image acquisition method, a plurality of image slices of the region of interest are acquired sequentially and the final image is composed during reconstruction of data of the individual scans. The step-and-shoot image acquisition may also be known as sequential axial image acquisition.

In an embodiment of the invention, the parameter of the image acquisition is a distance between two individual successive scans of the step-and-shoot image acquisition. Thus, the couch travel adjusting the increment value between two successive axial scans, i.e., shots, can be adjusted in order to avoid gaps between adjacent image frames.

In an embodiment of the invention, the measurement data about a movement of the patient to be imaged are received between two individual successive scans of the step-and-shoot image acquisition. However, the measurement data can also be acquired during the individual scans.

In an embodiment of the invention, the distance between the two individual successive scans of the step-and-shoot image acquisition is adapted based on the determined motion such that an overlap between the two individual successive scans occurs without any gap in between the two individual successive scans of the step-and-shoot image acquisition.

In an embodiment of the invention, the image acquisition is a helical image acquisition.

In an embodiment of the invention, the parameter of the image acquisition is a pitch of the helical image acquisition. Thus, the image acquisition can be adapted by reducing the pitch of the helical scan in order to increase the overlapping regions and to avoid gaps in the image acquisition.

In an embodiment of the invention, the method comprises further the step of reconstructing an image of at least a region of the patient, the step of reconstruction the image comprising the step of correcting data acquired in the image acquisition for the determined motion of the patient.

In an embodiment of the invention, the method comprises the step of triggering the acquisition of the image when the movement of the patient has stopped. Thus, the trigger time can be regarded as the parameter of the image acquisition that is adapted according to the detected patient motion.

In an embodiment of the invention, the method comprises the step of re-acquiring a previously acquired image of at least a region of the patient with the computed tomography apparatus in case it is determined that a movement of the patient occurred during acquisition of the previously acquired image. Thus, an initial parameter value may be to acquire the next image frame at a couch position a few centimeters further down, but the adapted value may be at the same position as the previous scan.

According to another aspect of the invention, there is provided a device for adapting a parameter of an image acquisition with a computed tomography apparatus to a motion of a patient to be imaged. The device comprises a motion sensor configured for receiving measurement data about a movement of a patient to be imaged with the computed tomography apparatus, and a data processing apparatus configured for determining, on the basis of the measurement data of the motion sensor, a motion of the patient, determining, in case the determined motion exceeds a predetermined threshold, an adapted value of the parameter of the image acquisition based on the determined motion, and initiating acquisition of an image of at least a region of the patient with the computed tomography apparatus utilizing the adapted value of the parameter of the image acquisition.

In an embodiment of the invention, the motion sensor is a contactless motion sensor.

In an embodiment of the invention, the motion sensor is an optical camera.

According to another aspect of the invention, there is provided a data processing apparatus comprising means for carrying out the steps of determining, on the basis of measurement data of a motion sensor about a movement of a patient to be imaged with a computed tomography apparatus, a motion of the patient, determining, in case the determined motion exceeds a predetermined threshold, an adapted value of a parameter of an image acquisition with the computed tomography apparatus based on the determined motion, and initiating acquisition of an image of at least a region of the patient with the computed tomography apparatus utilizing the adapted value of the parameter of the image acquisition.

According to another aspect of the invention, there is provided a computed tomography apparatus comprising the device according to any of the preceding embodiments.

In summary, the invention relates to a method for adapting a parameter of an image acquisition with a computed tomography apparatus to a motion of a patient to be imaged. The method comprises the steps of receiving, with a motion sensor, measurement data about a movement of a patient to be imaged with the computed tomography apparatus, and determining, on the basis of the measurement data of the motion sensor, a motion of the patient. The method comprises further the steps of determining, in case the determined motion exceeds a predetermined threshold, an adapted value of the parameter of the image acquisition based on the determined motion, and acquiring an image of at least a region of the patient with the computed tomography apparatus utilizing the adapted value of the parameter of the image acquisition.

One of the advantages of embodiments of the present invention is that a gap between subsequent image frames of an axial image acquisition of a computed tomography apparatus can be effectively avoided. Another advantage may reside in that motion artifacts in computed tomography imaging can be avoided. Another advantage may be that image reconstruction is enabled to more reliably reconstruct images when patient motion has occurred.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a block diagram of a method for adapting a parameter of an image acquisition with a computed tomography apparatus to a motion of a patient to be imaged according to an embodiment of the invention.
Fig. 2 shows an illustration of a step-and-shoot computed tomography image acquisition that is deteriorated by a movement of the patient's head during image acquisition.
Fig. 3 shows an illustration of a motion of the patient's head during imaging that is compensated for in the image reconstruction algorithm.
Fig. 4 shows a schematic setup of a computed tomography apparatus comprising the device for adapting a parameter of an image acquisition with a computed tomography apparatus to a motion of a patient to be imaged according to an embodiment of the invention.

### DETAILED DESCRIPTION OF EMBODIMENTS

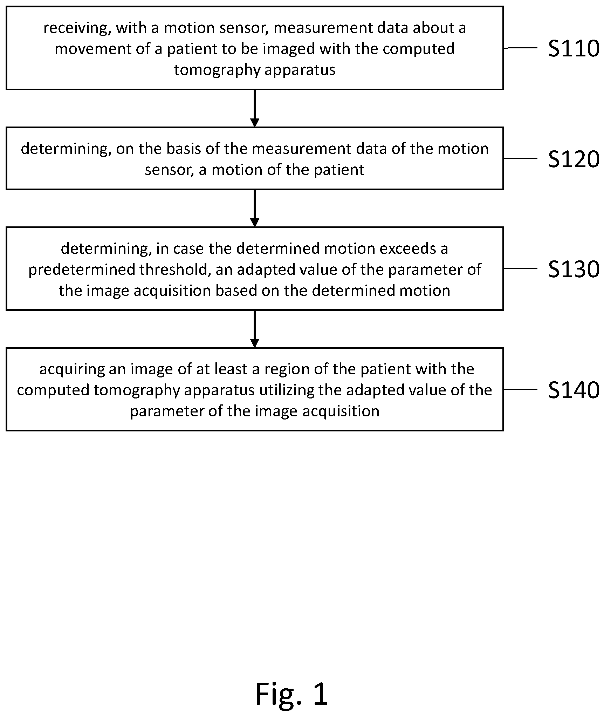

Fig. 1 shows a block diagram of a method for adapting a parameter of an image acquisition 160 with a computed tomography apparatus 200 to a motion of a patient 110 to be imaged according to an embodiment of the invention. The method comprises the step S110 of receiving, with a motion sensor 120, measurement data 130 about a movement 170 of a patient 110 to be imaged with the computed tomography apparatus 200, and the step S120 of determining, on the basis of the measurement data 130 of the motion sensor 120, a motion of the patient 110. The method comprises further the step S130 of determining, in case the determined motion exceeds a predetermined threshold, an adapted value of the parameter 140 of the image acquisition 160 based on the determined motion, and the step S 140 of acquiring S140 an image of at least a region of the patient 110 with the computed tomography apparatus 200 utilizing the adapted value of the parameter 140 of the image acquisition 160.

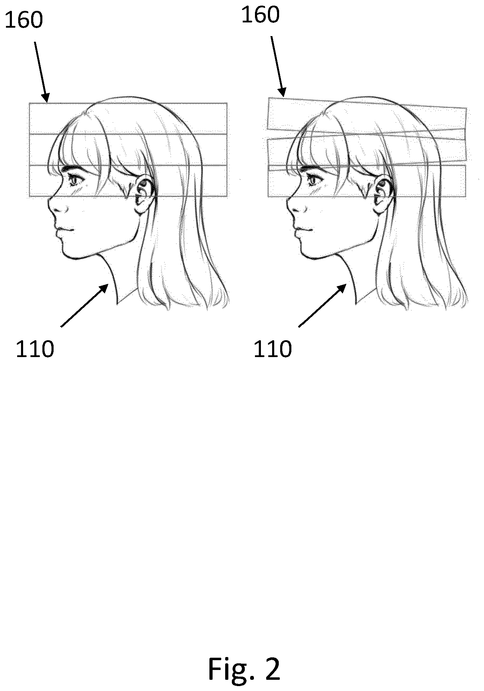

Fig. 2 shows an illustration of a step-and-shoot computed tomography image acquisition that is deteriorated by a movement 170 of the patient's 110 head during image acquisition 160. The left picture of Fig. 2 illustrates the desired scan and the desired coverage of three sequential axial computed tomography scans in the patient's head coordinate system. However, there may be still motion of the patient 110 present between the individual axial scans of the image acquisition 160. This is illustrated in the right picture of Fig. 2. Here it is shown how the scanned area by the three axial scans changes if the head was in the planned position for the lowermost scan, but the scanned area of the patient 110 changed due to a nodding motion for the middle scan of about +3 degrees and of about -3 degrees for the top scan. Apparently, gaps in the image acquisition 160 occur, which is undesired.

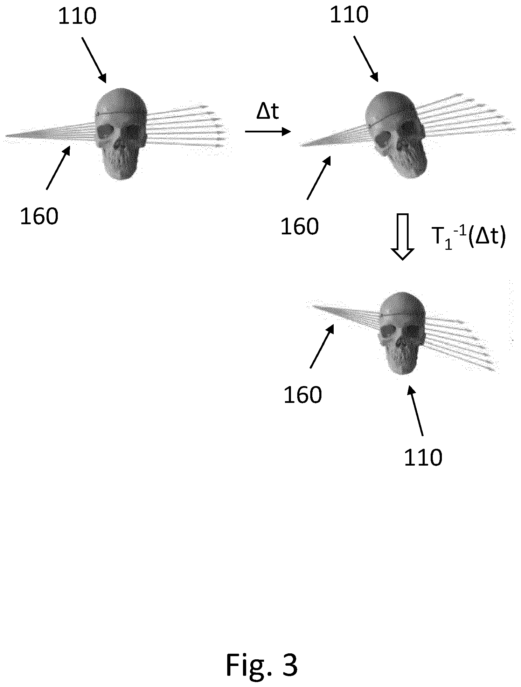

Fig. 3 shows an illustration of a motion of the patient's 110 head during imaging that is compensated for in the image reconstruction algorithm. While the head computed tomography scan is acquired, the patient's head can be tracked by a contactless motion sensor such as an optical camera. The computation of the head motion parameters such as rotation and translation from camera data can be done in several ways. For example, a depth camera, like a 3D camera, can be used in order to measure directly the position of the head surface at successive time points. Alternatively, a regular 2D video camera, colored or infrared, for example, can be used, which may have the advantage of higher spatial and temporal resolution. In this case, the full head motion parameters can be obtained by tracking specific head landmarks in 2D and then solving the inverse problem that consists in computation the 3D rotation and translation that will minimize the residual landmark displacement. This latter approach can be refined by the use of a 3D head surface model. This 3D head surface model can be a template or a subjectspecific model acquired with a dedicated 3D camera. Preferably, the optical camera is calibrated with respect to the scanner geometry, so that the geometry transformation between camera and scanner coordinates is known. In this way, the motion parameters obtained from the camera data can be transformed into the scanner coordinates where they can be applied as described below. In the following, all geometric considerations will be described between a first and a second projection of the computed tomography image acquisition 160 process. The same principles apply for all further projections acquired during the head examination. In the first image on the upper left, an initial position Po of the head is shown. Any rigid motion of the head can be described by six parameters, i.e. three angles describing the rotation of the head and three parameters describing the translation of the head. The motion of the head of the subject 110 in the time span Δt between the image acquisition 160 of the first projection at time to and the second projection at time ti is described by the transformation T₁. T₁ can be derived from the motion sensor, e.g., by standard computer vision methods. In the second image on the upper right, the rotated head of the patient is shown, together with an indication of a gantry movement between the first and the second projection. Standard reconstruction methods expect the target object like the patient's head to remain unaltered. Head motion will lead to motion artefacts if not explicitly accounted for. In order to compensate for this head motion of the patient 110, the x-ray projection geometry of the computed tomography apparatus can be corrected by applying the inverse transform T₁⁻¹, which is shown in the picture on the lower right of Fig. 3. At the end of the scan, each forward projection is corrected to compensate the head motion, resulting in a set of projection data, each of which is equipped with its individual projection geometry. This does not follow the traditional axial or helical scanning geometry. To reconstruct the CT volume, a reconstruction method capable of handling arbitrary projection geometries is applied, such as iterative reconstruction methods.

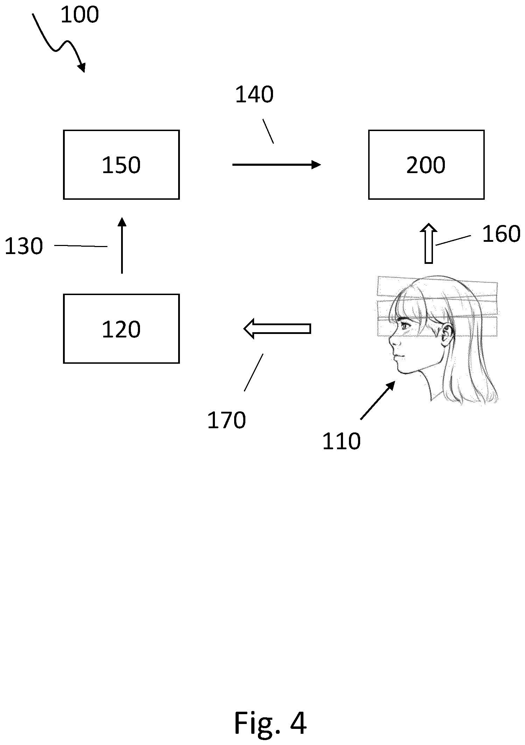

Fig. 4 shows a schematic setup of a computed tomography apparatus 200 comprising the device 100 for adapting a parameter of an image acquisition 160 with a computed tomography apparatus 200 to a motion of a patient 110 to be imaged according to an embodiment of the invention. A movement 170 of a patient 110 to be imaged with the computed tomography apparatus 200 is received with a motion sensor 120, which is preferably a contactless motion sensor like an optical camera, or an infrared camera. Measurement data 130 about the received movement 170 are forwarded to a processing unit 150. Based on the measurement data 130 of the motion sensor 120, a motion of the patient 110 is determined. In case the determined motion of the patient 110 exceeds a predetermined threshold, an adapted value of the parameter 140 of the image acquisition 160 is generated based on the determined motion and forwarded to the computed tomography apparatus 200. For example, in case the image acquisition 160 is a sequential axial image acquisition, the adapted value of the parameter 140 of the image acquisition 160 can be a distance between two individual successive scans of the sequential axial image acquisition, such that the distance between the two individual successive scans of the sequential axial image acquisition is adapted based on the determined motion such that an overlap between the two individual successive scans occurs without any gap in between the two individual successive scans of the sequential axial image acquisition. Otherwise, in case the image acquisition 160 is a helical image acquisition, the adapted value of the parameter 140 of the image acquisition 160 can be a pitch of the helical image acquisition. In addition or alternatively, an image acquisition can be triggered when a possible movement 170 of the patient 110 has stopped. In this case, the adapted value of the parameter 140 of the image acquisition 160 refers to the trigger time of the acquisition of the next image. Further, a previously acquired image can be re-acquired with the computed tomography apparatus 220 in case it is determined that a movement 170 of the patient 110 occurred during acquisition of the previously acquired image. In this case, the adapted value of the parameter 140 of the image acquisition 160 refers to the position of the acquisition of the next subsequent image frame. As a next step, an image of at least a region of the patient 110 is acquired with the computed tomography apparatus 200 utilizing the adapted value of the parameter 140 of the image acquisition 160.

Optionally, in the reconstruction phase of reconstructing an image of at least a region of the patient 110, data acquired in the image acquisition 160 can be corrected for the determined motion of the patient 110 as already described with reference to Fig. 3.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: device for adapting a parameter
- 110: patient
- 120: motion sensor
- 130: measurement data
- 140: adapted value of the parameter
- 150: data processing apparatus
- 160: image acquisition
- 170: movement
- 200: computed tomography apparatus

## Claims

1. A method for adapting a parameter of an image acquisition (160) with a computed tomography apparatus (200) to a motion of a patient (110) to be imaged, the method comprising the steps of:
receiving (S110), with a motion sensor (120), measurement data (130) about a movement (170) of a patient (110) to be imaged with the computed tomography apparatus (200);
determining (S120), on the basis of the measurement data (130) of the motion sensor (120), a motion of the patient (110);
determining (S 130), in case the determined motion exceeds a predetermined threshold, an adapted value of the parameter (140) of the image acquisition (160) based on the determined motion; and
acquiring (S140) an image of at least a region of the patient (110) with the computed tomography apparatus (200) utilizing the adapted value of the parameter (140) of the image acquisition (160).

2. The method according to claim 1, wherein the image acquisition (160) is a step-and-shoot image acquisition.

3. The method according to claim 2, wherein the parameter of the image acquisition (160) is a distance between two individual successive scans of the step-and-shoot image acquisition.

4. The method according to claim 3, wherein the measurement data (130) about a movement (170) of the patient (110) to be imaged are received between two individual successive scans of the step-and-shoot an image acquisition.

5. The method according to any of claims 3 or 4, wherein the distance between the two individual successive scans of the step-and-shoot image acquisition is adapted based on the determined motion such that an overlap between the two individual successive scans occurs without any gap in between the two individual successive scans of the step-and-shoot image acquisition.

6. The method according to claim 1, wherein the image acquisition (160) is a helical image acquisition.

7. The method according to claim 6, wherein the parameter of the image acquisition (160) is a pitch of the helical image acquisition.

8. The method according to any of the preceding claims, the method comprising further the step of reconstructing an image of at least a region of the patient (110), the step of reconstruction the image comprising the step of correcting data acquired in the image acquisition (160) for the determined motion of the patient (110).

9. The method according to any of the preceding claims, the method comprising the step of triggering the acquisition of the image when the movement (170) of the patient (110) has stopped.

10. The method according to any of the preceding claims, the method comprising the step of re-acquiring a previously acquired image of at least a region of the patient (110) with the computed tomography apparatus (220) in case it is determined that a movement (170) of the patient (110) occurred during acquisition of the previously acquired image.

11. A device (100) for adapting a parameter of an image acquisition (160) with a computed tomography apparatus (200) to a motion of a patient (110) to be imaged, the device (100) comprising
a motion sensor (120) configured for receiving measurement data (130) about a movement (170) of a patient (110) to be imaged with the computed tomography apparatus (200); and
a data processing apparatus (150) configured for
determining, on the basis of the measurement data (130) of the motion sensor (120), a motion of the patient (110),
determining, in case the determined motion exceeds a predetermined threshold, an adapted value of the parameter (140) of the image acquisition (160) based on the determined motion, and
initiating acquisition of an image of at least a region of the patient (110) with the computed tomography apparatus (200) utilizing the adapted value of the parameter (140) of the image acquisition (160).

12. The device according to claim 11, wherein the motion sensor (120) is a contactless motion sensor.

13. The device according to any of claims 11 or 12, wherein the motion sensor (120) is an optical camera.

14. A data processing apparatus (150) comprising means for carrying out the steps of
determining, on the basis of measurement data (130) of a motion sensor (120) about a movement (170) of a patient (110) to be imaged with a computed tomography apparatus (200), a motion of the patient (110),
determining, in case the determined motion exceeds a predetermined threshold, an adapted value of a parameter (140) of an image acquisition (160) with the computed tomography apparatus (200) based on the determined motion, and
initiating acquisition of an image of at least a region of the patient (110) with the computed tomography apparatus (200) utilizing the adapted value of the parameter (140) of the image acquisition (160).

15. A computed tomography apparatus (200) comprising the device (100) according to any of claims 11 to 13.
